# EUROPÄISCHE PATENTSCHRIFT

(11) **EP 2 524 711 B1**
(45) Veröffentlichungstag und Bekanntmachung des Hinweises auf die Patenterteilung: **08.01.2014**
(21) Anmeldenummer: 11075089.0
(22) Anmeldetag: 16.05.2011
(51) Int. Cl.: A61M 1/10, A61M 1/36, A61M 39/10, A61M 39/12, F16L 37/10

(54) **Verbindungssystem zur Herstellung eines Verbindungskanals für Körperflüssigkeiten**
Connection system for producing a connection channel for bodily fluids
Système de liaison pour la fabrication d'un canal de liaison pour liquides corporels

(43) Veröffentlichungstag der Anmeldung: 21.11.2012
(73) Patentinhaber: Berlin Heart GmbH, 12247 Berlin (DE)
(72) Erfinder: Göllner, Manfred, 13086 Berlin (DE); Samlenski, Uwe, 12169 Berlin (DE)
(74) Vertreter: Pfenning, Meinig & Partner GbR

(56) Entgegenhaltungen:
- EP-B1- 1 516 142
- DE-A1-102009 047 844
- GB-A- 2 407 593
- US-A- 5 868 437
- US-A1- 2007 197 855

## Beschreibung

Die Erfindung liegt auf dem Gebiet der Mechanik und bezieht sich auf die Herstellung von fluiddichten Verbindungskanälen, die teilweise aus Schläuchen, Rohren, Stutzen und anderen Elementen, die Durchgangsöffnungen aufweisen, bestehen können. Die Erfindung ist unter anderem speziell auf dem Gebiet der Medizintechnik anwendbar und kann dort der einfachen Herstellung einer Verbindung zwischen verschiedenen Leitungs- und Rohrelementen dienen.

Besonders bei Operationen, die mit dem Blutkreislaufsystem im menschlichen Körper zu tun haben, beispielsweise Operationen an Blutgefäßen, ist es oft nötig, neue Verbindungskanäle zur Leitung des Blutes herzustellen. Oft wird zu diesem Zweck ein erstes, etwas biegbares, aber nicht radial aufdehnbares schlauchförmiges Element, auch Graft genannt, direkt mit einem Gefäß verbunden. Dieses erste Element muss dann möglichst flexibel mit einem weiterführenden Leitungselement verbunden werden können, ohne dass dazu aufwendige Werkzeuge benötigt werden. Die Verbindung muss dicht und zuverlässig sein und darf in der Blutströmung keine Totwassergebiete schaffen, in denen sich Blut sammeln und koagulieren könnte.

Die Medizintechnik hat sich bereits grundsätzlich mit Verbindungssystemen beschäftigt. So ist beispielsweise aus der US-Patentschrift 6 942 672 B2 ein Verbindungssystem mit einem Graft bekannt, das einerseits mit einem Blutgefäß und am gegenüberliegenden Ende mit einem Leitungselement verbindbar ist.

Aus der EP 1 516 142 B1 ist ein Verbindungssystem für eine Kanüle und ein Rohr bekannt, das ineinandersteckbare Elemente sowie eine axiale Halteeinrichtung der Verbindung mit Schnappriegeln aufweist.

Aus der DE 37 15 911 C2 ist eine Vorrichtung zur Verbindung einer Schlauchspitze mit einem Schlauchende eines extrakorporalen medizinischen Versorgungssystems bekannt.

Der vorliegenden Erfindung liegt vor dem Hintergrund des genannten Standes der Technik die Aufgabe zugrunde, ein Verbindungssystem zu schaffen, das eine möglichst einfache Verbindung von Leitungselementen bei möglichst unaufwendiger Konstruktion ermöglicht, wobei die Verbindung zuverlässig flüssigkeitsdicht ist und das Zusammenfügen verschiedener Elemente erst kurz vor oder während einer Operation mit einfachen Mitteln erlauben soll.

Die Aufgabe wird mit den Merkmalen der Erfindung gemäß Patentanspruch 1 gelöst.

Hierzu ist bei einem Verbindungssystem zur Herstellung einer Gefäßkanüle mit einem flexiblen Schlauchelement, insbesondere einem radial nicht dehnbaren Graft, einem Leitungselement sowie einem Verbindungselement zur flüssigkeitsdichten Verbindung mit dem Schlauchelement einerseits und dem Leitungselement andererseits vorgesehen, dass das Verbindungselement sowohl mit dem Schlauchelement als auch mit dem Leitungselement axial überlappt und dass ein Halteelement mit einem radial elastisch aufweitbaren Schnappring vorgesehen ist, der radial nach außen über andere Teile des Halteelementes umbördelbar ist und der im Verbindungszustand auf ein Element des Verbindungssystems aufschnappbar ist.

Das Schlauchelement, das Leitungselement und das zwischen diesen liegende Verbindungselement sind dabei axial zusammensteckbar und vorteilhaft jeweils mittels einer Radialdichtung gedichtet. Ein einfaches Zusammenstecken ist insofern vorteilhaft, als das Schlauchelement, das Verbindungselement und das Leitungselement aus unterschiedlichen Materialien bestehen können und unterschiedlichen Desinfektions- und/oder Lagerungsvorschriften unterliegen. Es ist vorteilhaft, die einzelnen Elemente des Verbindungssystems getrennt voneinander lagern und erst kurz vor oder während der Operation zusammenführen zu können. Die einzelnen Teile können zudem von unterschiedlichen Herstellern bezogen werden. Wird während der Operation der Austausch eines der Teile, beispielsweise bei einer Fehlkürzung des Grafts, notwendig, so können die übrigen Teile problemlos weiterverwendet und das auszutauschende Teil kann einzeln ersetzt werden.

Das flexible Schlauchelement ist als Graft, üblicherweise als wellenrohrartiges oder spiralverstärktes schlauch- oder rohrförmiges Gebilde ausgeführt, das oft aus Polyestergewebe oder PTFE besteht. Dieses Schlauchelement dient oft als Ersatz für ein Blutgefäß und wird in der Regel direkt mit dem Gefäß vernäht oder aber ist mittels einer flanschartigen Vorrichtung, wie beispielsweise eines Nahtrings oder einer ähnlichen Konstruktion, mit einem Blutgefäß oder einer Herzwand flüssigkeitsdicht verbindbar.

Ein Graft sollte in gewissem Maß flexibel sein, um sich anatomischen Gegebenheiten anpassen zu können und um die Einwirkung von Kräften auf Blutgefäße zu begrenzen. Aus diesem Grund ist ein Graft üblicherweise flexibel zu allen Seiten biegbar. Er kann jedoch derart konstruiert sein, dass er beispielsweise trotz dieser Flexibilität radial praktisch nicht aufweitbar ist. Hierdurch werden besondere Anforderungen an die Anschließbarkeit von Fortsetzungsleitungselementen an einen Graft gestellt, beispielsweise wenn diese in den Graft einsteckbar sein sollen. Die erfindungsgemäße Verbindungstechnik mit dem mehrteiligen System erlaubt in diesem Zusammenhang das Herstellen einer zuverlässig dichten lösbaren Verbindung bei gleichzeitig guter Handhabbarkeit während der Operation und beim Zusammenfügen des Verbindungssystems.

Beispielsweise kann das Verbindungselement aus einem Metall, insbesondere Titan, bestehen und ist dann praktisch nicht verformbar. Der Graft und das Verbindungselement sollten vorteilhaft bezüglich ihrer Durchmesser genau aufeinander angepasst sein. Der Graft kann auch teilweise am Ende geschlitzt werden, um ihn über einen vorstehenden Bund auf das Verbindungselement aufzuschieben und hinter dem Bund auf dem Verbindungselement durch eine Fadenwicklung zu fixieren. Das Leitungselement kann beispielsweise aus Polyurethan oder Silikon bestehen und auch elastisch aufweitbar und damit relativ einfach auf das Verbindungselement aufschiebbar oder in dieses einführbar sein.

Grundsätzlich hat das erfindungsgemäße Halteelement mit dem aufweitbaren Schnappring die Funktion, den axialen Zusammenhang des Verbindungssystems herzustellen und zu sichern. Zu diesem Zweck ist üblicherweise das Halteelement mit einem Element des Verbindungssystems fest verbunden, und der Schnappring des Halteelementes kann hinter einer Rastfläche eines anderen Teils des Verbindungssystems derart eingeschnappt werden, dass der axiale Zusammenhalt hergestellt wird.

Zudem kann der Durchmesser und die Aufweitbarkeit des Schnapprings derart gestaltet sein, dass dieser im Verbindungszustand nach dem Einschnappen hinter der Rastfläche auf das Element des Verbindungssystems, auf das er aufgeschnappt ist, auch in radialer Richtung eine Kompressionswirkung ausübt und somit in diesem Bereich zwei Dichtungspartner, die dort mit einer Radialdichtung dichten, zusammenpresst. Hierdurch wird dann nicht nur der axiale Zusammenhalt, sondern auch die radiale Dichtung der einzelnen Teile des Verbindungssystems unterstützt und gesichert.

Das Halteelement kann vorteilhaft mit dem Leitungselement verbunden sein, insbesondere auch einstückig mit diesem zusammenhängen. Dabei kann das Leitungselement auch unmittelbar als ein Pumpenanschluss ausgebildet sein. Es kann beispielsweise einstückig mit Teilen der Pumpe zusammenhängen.

Der Schnappring des Halteelementes kann auf diese Weise beispielsweise hinter einen azimutal umlaufenden Steg des Verbindungselementes oder des flexiblen Schlauchelementes einschnappen, um den axialen Zusammenhang herzustellen. Der Schnappring kann beispielsweise auch in eine entsprechende Nut an einem mit dem Leitungselement zu verbindenden Element einschnappen.

Ist das Leitungselement beispielsweise aus einem elastischen Material, z.B. einem Silikonelastomer, hergestellt, so kann das Halteelement einstückig mit diesem und aus demselben Material angeformt sein. Das Halteelement kann als eine Art Filmgelenk ausgebildet sein, das an der äußeren Mantelfläche des Leitungselementes befestigt ist und von dieser ausgehend ein Schlauchstück bildet, das das Leitungselement koaxial umgibt und in dem Schnappring endet.

Das Halteelement kann auch beschrieben werden als ein außen an dem Leitungselement umlaufender Flansch, an dem ein koaxial zu dem Leitungselement verlaufendes Rohrstück befestigt ist, das in dem Schnappring endet, wobei das Rohrstück wenigstens einen Schwächungsbereich aufweist, in dem seine Wand azimutal umlaufend derart geschwächt ist, dass das Schlauchstück dort insgesamt umbördelbar ist. Durch dieses Umbördeln wird der Schnappring aufgeweitet und hinter andere Teile des Rohrstücks bzw. des Halteelementes zurückgeklappt. In dieser Position kann das Halteelement selbststabilisierend sein, so dass der Schnappring beispielsweise vor einer Operation vorgespannt werden kann und dann zum Herstellen einer Verbindung des Verbindungssystems mit nur noch relativ geringem Aufwand zum Ende des Leitungselements umgeklappt werden muss, um hinter einer Rastfläche eines Verbindungspartners einzuschnappen.

Das Halteelement kann auch direkt mit dem Verbindungselement verbunden sein und insbesondere einstückig mit diesem zusammenhängen. In diesem Fall wird das Halteelement aus einem anderen Material bestehen müssen als das Verbindungselement, damit es ausreichend elastisch verformbar ist. Ansonsten kann das Halteelement auch einfach mit üblichen Fügetechniken mit dem Verbindungselement zusammengefügt sein, beispielsweise durch Kleben, Angießen oder ähnliche Verfahren. Das Halteelement kann dann durch entsprechendes Aufschnappen des Schnapprings entweder mit dem Leitungselement einerseits oder mit dem flexiblen Schlauchelement andererseits verbunden werden.

Es können auf dem Verbindungselement auch mehrere Halteelemente vorgesehen sein, von denen jeweils eines in Richtung des Leitungselementes und eines in Richtung des Schlauchelementes weist. Auf diese Weise kann vor einer Operation zuerst die Verbindung zwischen dem Verbindungselement und dem Leitungselement und später dann die Verbindung zu dem Schlauchelement hergestellt werden.

Es kann vorteilhaft zudem vorgesehen sein, dass das Verbindungselement in das Schlauchelement axial einschiebbar ist und sich im Verbindungszustand radial innerhalb des Schlauchelementes befindet.

Das Schlauchelement muss dazu bezüglich des Durchmessers genau zu dem Verbindungselement passen. Zusätzlich kann ein beide Dichtungspartner von radial außen umfassendes Druckelement vorgesehen sein, das die Dichtungspartner dieser Radialdichtung zusammenpresst. Hierdurch wird das Unterbluten dieser Dichtung verhindert, das zu einer ungewollten Speicherung von geringen Blutmengen im Falle des Einsatzes bei einem Blutgefäß oder von anderen Körperflüssigkeiten führt und das vermieden werden muss, um dort nicht Zerfallsprozesse oder andere Prozesse der Körperflüssigkeiten, wie z.B. Blutkoagulation, unkontrolliert stattfinden zu lassen.

Auch in das Leitungselement kann das Verbindungselement axial einschiebbar sein. Das Leitungselement kann hierzu elastisch aufweitbar sein, und es kann auch an dieser Stelle zur Unterstützung ein die Dichtungspartner der Radialdichtung umfassendes Druckelement für den radialen Druckaufbau vorgesehen sein.

Ein weiteres Sicherungselement für den axialen Zusammenhalt kann vorteilhaft beispielsweise durch eine Fadenwicklung gebildet sein. Hierzu kann ein Faden um den äußeren Dichtungspartner mehrfach herumgewickelt werden, beispielweise auch spiralig, um einen Radialdruck aufzubauen. Dafür sollte das äußere Element der Radialdichtung zumindest ein Stück weit radial elastisch verformbar sein, um ein Aufpressen auf den inneren Dichtungspartner zu erlauben. Als Wickelfaden kann vorteilhaft ein Operationsnähfaden verwendet werden.

Es kann hierzu im Bereich der Fadenwicklung oder allgemeiner des Druckelementes auch eine Wandschwächung des äußeren Dichtungspartners, also beispielsweise eine Schwächung der Wand des Schlauchelementes oder Leitungselementes, vorgesehen sein.

Das Verbindungselement kann zur Herstellung einer zuverlässigen Dichtung beispielsweise auch radial umlaufende Stege auf seiner Außenseite aufweisen, die im Längsschnitt eine Sägezahnstruktur haben können. Diese an sich bekannte Struktur einer Schlauchkupplung schafft eine sichere Verbindung und eine Gewährleistung hoher Dichtigkeit. Außerdem entsteht hierdurch eine mehrstufige Dichtung, deren Zuverlässigkeit durch das Aufpressen eines Druckelementes von außen noch erhöht wird.

Das äußere Druckelement kann beispielsweise auch durch den Schnappring selbst gebildet sein, so dass dieser den entsprechenden radialen Druck zur Unterstützung der Dichtung ausübt. Hierzu ist es vorteilhaft, wenn der Schnappring im entspannten Zustand einen geringeren Innendurchmesser aufweist als der Außendurchmesser des Elementes, auf das er aufgeschnappt wird.

Eine konkrete Variante der Verbindung zweier Elemente des Verbindungssystems sieht vorteilhaft vor, dass diese, beispielsweise das flexible Schlauchelement und das Verbindungselement, mittels eines umlaufenden Gewindes zusammenschraubbar sind. Dies bietet sich insbesondere im Bereich des flexiblen Schlauchelementes an, wenn dies als Graft ausgebildet ist, der an sich schon eine gewindeartige Struktur bildet. Eine solche Zentralgewindeverbindung sorgt zumindest für den axialen Zusammenhalt der Anordnung. Wird der Schnappring derart ausgebildet und positioniert, dass er von außen auf den äußeren Partner dieser Schraubverbindung einwirkt und diesen radial zusammenpresst, so kann hierdurch eine Erhöhung des Zusammenhalts und der Haftreibung sowie die Dichtigkeit der Radialdichtung erreicht werden.

Im Folgenden werden weitere Konkretisierungen und vorteilhafte Ausgestaltungen der Erfindung anhand eines Ausführungsbeispiels in einer Zeichnung gezeigt und anschließend beschrieben.

Dabei zeigt
- Fig. 1: schematisch drei Teile eines Verbindungssystems in einem Längsschnitt,
- Fig. 2: ein Leitungselement mit einem Halteelement, im oberen Teil mit umgebördeltem Schnappring, im unteren Teil im Verbindungszustand,
- Fig. 3a: ein flexibles Schlauchelement mit einem mit diesem fest (unlösbar)verbundenen Verbindungselement,
- Fig. 3b: ein vergrößertes Teil aus Fig. 3a,
- Fig. 4: im Längsschnitt ein Schlauchelement/Graft, das mit einem Verbindungselement verbunden ist, vor dem Einführen in ein Leitungselement,
- Fig. 5: im Längsschnitt ein Verbindungssystem im zusammengefügten Zustand / Verbindungszustand,
- Fig. 6: ein Verbindungssystem, bei dem das Halteelement mit dem Leitungselement verbunden ist und das Verbindungselement ein umlaufendes Gewinde zur Verbindung mit dem Schlauchelement aufweist, sowie
- Fig. 7: die Konstellation aus Fig. 6 im Verbindungszustand.

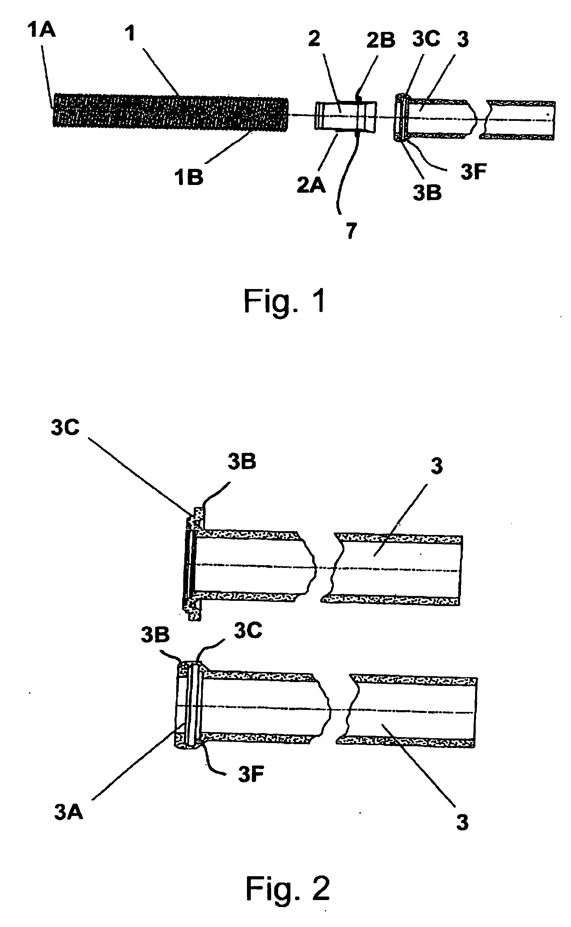

**Figur 1** zeigt in einem Längsschnitt hintereinander das Verbindungssystem mit einem flexiblen Schlauchelement 1, ausgeführt als Graft, sowie einem Leitungselement 3, das als Rohr aus einem Silikon oder Silikonelastomer ausgeführt ist. Außerdem ist das Verbindungselement 2 dargestellt, mit einem azimutal umlaufenden Steg, der eine Rastfläche 2B bildet. Die drei Elemente des Verbindungssystems sind axial hintereinander und koaxial zueinander angeordnet.

Der Graft besteht aus einem Polyestergewebe, kann jedoch auch aus einem anderen Kunststoff gefertigt sein. Das Leitungselement 3 kann anstatt aus Silikon auch aus einem Polyurethan bestehen. Das Verbindungselement 2 besteht vorteilhaft aus einem Metall, insbesondere aus Titan.

Vorteilhaft ist das Verbindungselement 2 entweder fest mit dem Leitungselement 3 oder aber mit dem Schlauchelement 1 verbunden. Die fest verbundenen Teile werden als feste Verbindung gemeinsam sterilisiert und verpackt, während das die Gesamtverbindung ergänzende Teil (Schlauchelement oder Leitungselement) gesondert sterilisiert und verpackt werden kann.

Das Verbindungselement 2 ist derart dimensioniert, dass seine beiden Enden in den Graft einerseits und in das Leitungselement andererseits eingeschoben werden können. Eine dieser Verbindungen wird allerdings üblicherweise bereits vorkonfektioniert.

Von den Enden 1A, 1B des Grafts 1 wird das erste Ende 1A üblicherweise mit einem Blutgefäß vernäht, während das zweite Ende 1B mit dem Verbindungselement 2 verbunden wird.

Das Leitungselement 3 weist an seinem dem Verbindungselement 2 zugewandten Ende ein Halteelement 3B, 3C, 3F auf, das über einen azimutal an der Zylindermantelfläche des Verbindungselementes 2 umlaufenden Steg 7 geklappt und an einer Rastfläche 2B eingeschnappt wird.

**Figur 2** zeigt im oberen Teil das Halteelement mit nach hinten umgebördeltem Schnappring 3B, während der untere Teil der Abbildung das Halteelement im Verbindungszustand darstellt. Das Halteelement 3B, 3C, 3F ragt im Verbindungszustand axial über den rohrförmigen Teil des Leitungselementes 3 hinaus. Das Halteelement weist hierzu einen konischen zylindrisch umlaufenden Bereich 3F eines Schlauchstutzens sowie einen Bereich 3C auf, in dem der Schlauchstutzen zylindrisch und mit gleich bleibendem Innen- und Außendurchmesser verläuft und in dem der Schlauchstutzen durch einen geschwächten Wandbereich gekennzeichnet ist. An diesen geschwächten Wandbereich schließt sich der Schnappring 3B mit verstärkter Wanddicke an, wobei die Stärke des Schnapprings danach dimensioniert wird, dass der Schnappring beispielsweise im Verbindungszustand noch eine angemessen große radiale Druckwirkung ausüben kann, dass er jedoch leicht genug elastisch aufweitbar ist, um eine Umbördelung des Halteelementes zu ermöglichen. Der Durchmesser des Schnapprings im eingeschnappten Zustand ist in der Fig. 2 mit 3A bezeichnet. Die elastische Aufweitbarkeit des Schnapprings kann außer durch die Materialstärke und die Auswahl des Materials auch durch gegebenenfalls eingebrachte Einschnitte und Ausnehmungen geeignet gesteuert werden. Besonders vorteilhaft ist es jedoch, wenn der Schnappring mit gleich bleibendem Querschnitt und einschnittfrei gestaltet ist.

Die Geometrie des Halteelementes ist vorteilhaft so gestaltet, dass dieses sich im umgebördelten Zustand des Schnapprings 3B selbst stabilisiert, so dass das Leitungselement in diesem Zustand sterilisiert, transportiert und vorrätig gehalten werden kann.

Der Innendurchmesser 3D des Schnapprings 3B kann im entspannten Zustand gleich groß gewählt werden wie der Innendurchmesser des Rohrteils des Leitungselementes 3.

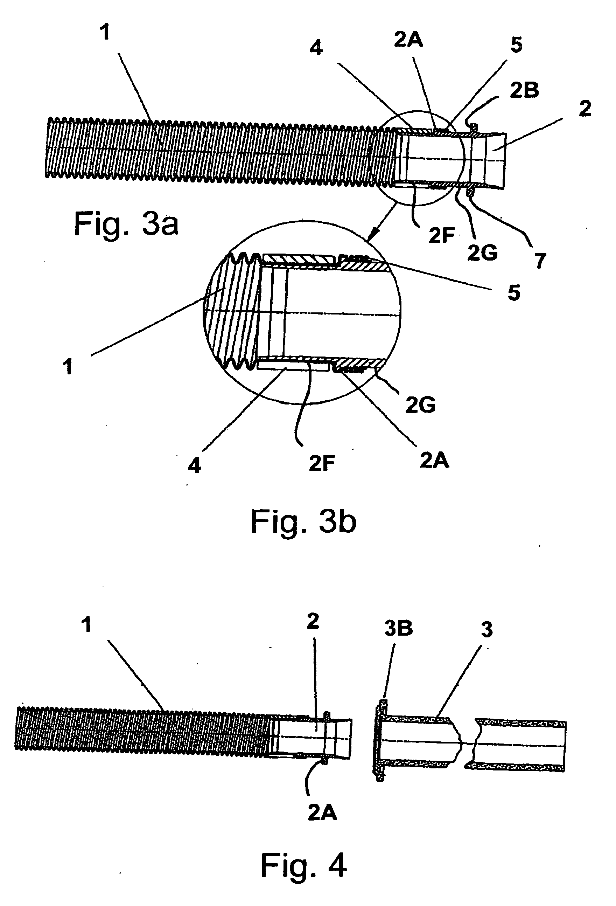

**Figur 3a** zeigt den Graft 1 in festen Verbindung mit einem Verbindungselement 2. Das Verbindungselement 2 weist einen Endabschnitt 2F und einen Mittenabschnitt 2G auf, die durch einen umlaufenden Bund oder eine Schulter 2A voneinander abgesetzt sind. Im Endabschnitt 2F weist das Verbindungselement einen geringeren Außendurchmesser auf als in dem Mittenabschnitt 2G.

Zur Verbindung wird das Ende 1B des Grafts 1 in dem Bereich, in dem dieser über den Bund 2A geschoben werden soll, geschlitzt und über das Verbindungselement 2 geschoben. Darauf wird der geschlitzte Längenbereich des Grafts 1 in dem mittleren Abschnitt 2G mit einer Fadenwicklung 5 umwickelt und gegen den mittleren Abschnitt 2G des Verbindungselementes 2 gepresst. Um in dem Endbereich 2F den dort aufliegenden Teil des Grafts 1 gegenüber dem Verbindungselement 2 zu dichten, ist dort eine geschlitzte ringförmige Hülse 4 aufgeschoben, die einen radialen Druck auf den Graft 1 ausübt und somit das Unterbluten der Radialdichtung zwischen dem Graft und dem Verbindungselement 2 in diesem Bereich verhindert und zudem den Graft fixiert. Der geschlitzte Ring 4 kann beispielsweise aus Titan bestehen. Es kann an dieser Stelle auch ein ungeschlitzter elastischer Ring, beispielsweise ein Silikonring, verwendet werden.

**Figur 3b** zeigt den Bereich des Endabschnitts 2F, des Bundes 2A und des Mittenabschnittes 2G in vergrößerter Darstellung.

**Figur 4** zeigt die vorgefertigte Verbindung zwischen dem Graft 1 und dem Verbindungselement 2, wie gemäß den Figuren 3a und 3b beschrieben, vor dem Zusammenfügen mit dem Leitungselement 3, das als Verbindungskanüle ausgebildet und in Fig. 4 im umgebördelten Zustand des Halteelementes mit dem aufgedehnten Schnappring 3B dargestellt ist.

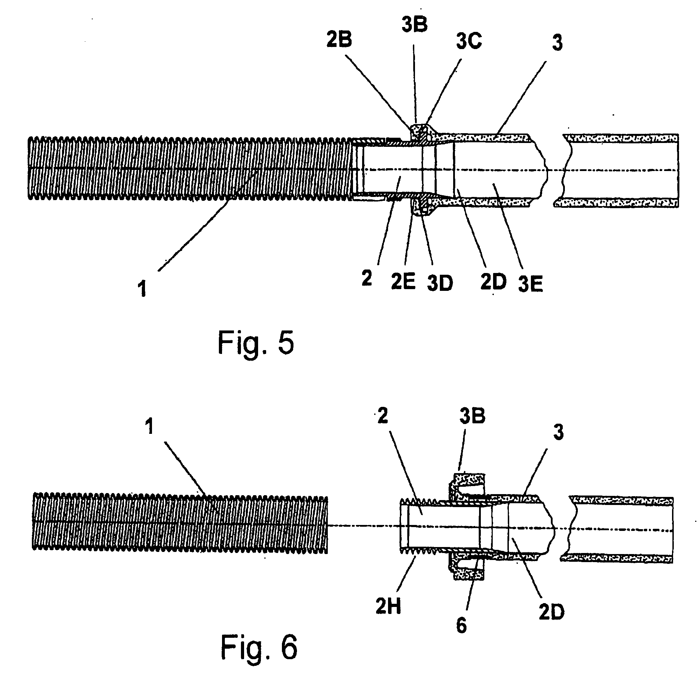

Das Verbindungselement 2 wird nun in das Leitungselement 3 eingeschoben, wie genauer in **Figur 5** dargestellt, und der Schnappring 3B wird hinter der Rastfläche 2A an dem außen azimutal umlaufenden Steg des Verbindungselementes 2 eingeschnappt.

Im Bereich 2E des Verbindungselementes 2, dargestellt in Fig. 5, weist dieses einen Außendurchmesser auf, der größer ist als der Innendurchmesser des Schnapprings 3B im entspannten Zustand. Dadurch kann in diesem Bereich auch der Graft, wenn er bis dort hinreicht, durch den Schnappring radial noch gegen das Verbindungselement gepresst und damit zusätzlich gehalten werden. Dort wird das Leitungselement 3 mit dem Verbindungselement zusätzlich zur Radialdichtung dieser Teile im Bereich 2D auch noch in einer Axialdichtung gedichtet, wo das Verbindungselement beispielsweise an seiner Außenseite noch eine sägezahnförmige Struktur von Außenringen aufweisen kann, um dort elastisch gegen das Leitungselement 3 gepresst zu werden. So kann auch verhindert werden, dass Flüssigkeit, insbesondere Blut, in den Bereich des Halteelementes 3B, 3C gelangt.

**Figur 6** zeigt eine abgewandelte Ausgestaltung der Erfindung, bei der an der Außenseite des Leitungselementes 3 noch ein geschwächter Wandbereich vor dem Halteelement 3B, 3C vorgesehen ist, in dem eine Fadenwicklung 6 angeordnet werden kann, um dort das Leitungselement 3 radial gegen das Verbindungselement 2 zu drücken und auf diese Weise den Halt zu sichern. Die Radialdichtung zwischen dem Verbindungselement und dem Leitungselement wird durch eine umlaufende Kante des Verbindungselementes, die an der Innenseite des elastischen Leitungselementes anliegt, gewährleistet.

Zudem ist an dem dem Graft zugewandten Ende des Verbindungselementes 2 ein Außengewinde 2H vorgesehen, das im Wesentlichen der wendelförmigen Struktur des Grafts 1 entspricht und auf das der Graft aufschraubbar ist. Hier wird die Dichtigkeit zusätzlich durch radialen Druck, beispielsweise mittels eines aufgebrachten Druckrings, hergestellt.

Die Dichtung des Verbindungselementes zum Leitungselement wird im Wesentlichen durch elastische Auflage des Leitungselementes auf der umlaufenden Kante 2D des Verbindungselementes bewirkt.

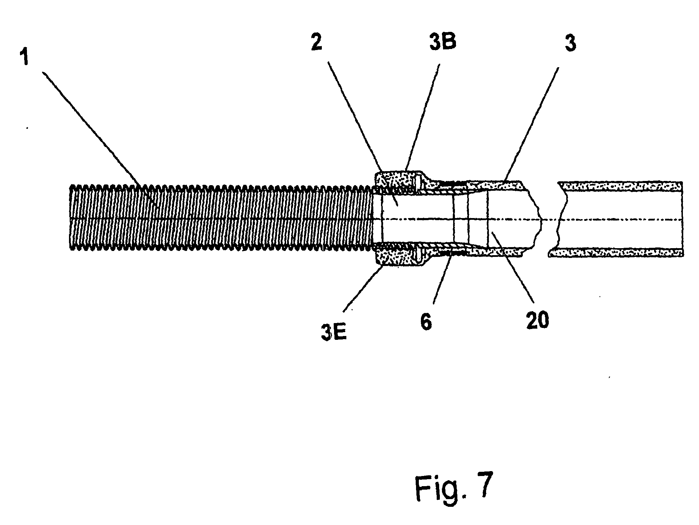

**Figur 7** zeigt für diesen Fall den Verbindungszustand, in dem der relativ massiv ausgestaltete Schnappring 3B radial auf den Graft im aufgeschraubten Zustand aufgeklappt ist, so dass der Graft im Bereich des Gewindes durch den Schnappring elastisch auf das Verbindungselement zur Vervollkommnung der Dichtung radial aufgedrückt wird. Gleichzeitig verhindert der elastisch aufgedrückte Schnappring 3B dort sowohl das axiale Abziehen des Grafts vom Verbindungselement 2 als auch ein Verdrehen, das zum Lösen des Gewindes führen könnte.

Die Erfindung ermöglicht in einfacher Weise die Herstellung einer zuverlässig dichten Verbindung eines Verbindungssystems mit drei Elementen, wobei sinnvollerweise das Verbindungselement bereits mit einem der anderen Elemente, idealerweise dem Leitungselement, vorkonfektioniert ist, so dass zwei Teile erst kurz vor der Operation zusammengefügt werden. Auf diese Weise ist der Bezug von verschiedenen Herstellern oder aus verschiedenen Lagerhaltungen und eine Kombination verschiedener passend auszuwählender Teile möglich. Auch wenn eines der Teile des Verbindungssystems während der Operation erneuert werden muss, können die übrigen Teile problemlos weiterverwendet werden.

Die Erfindung liefert dadurch einen Beitrag zur Vereinfachung von Operationen, bei denen Gefäßkanülen hergestellt werden müssen, wobei auch eine optimierte Beschaffung und Bevorratung ermöglicht ist.

## Patentansprüche

1. Verbindungssystem zur Herstellung einer Gefäßkanüle zum Anschluss an ein Blutgefäß mit einem flexiblen Schlauchelement (1), insbesondere einem Graft, einem Leitungselement (3) sowie einem Verbindungselement (2) zur flüssigkeitsdichten Verbindung mit dem Schlauchelement einerseits und dem Leitungselement andererseits, wobei das Verbindungselement sowohl mit dem Schlauchelement als auch mit dem Leitungselement axial überlappt und wobei ein Halteelement (3B, 3C, 3F) mit einem radial elastisch aufweitbaren Schnappring (3B) vorgesehen ist, der radial nach außen über andere Teile (3C) des Halteelementes umbördelbar ist und der im Verbindungszustand auf ein Element des Verbindungssystems aufschnappbar ist.

2. Verbindungssystem nach Anspruch 1, **dadurch gekennzeichnet, dass** das Halteelement (3B, 3C, 3F) mit dem Leitungselement (3) verbunden, insbesondere einstückig verbunden ist.

3. Verbindungssystem nach Anspruch 1 oder 2, **dadurch gekennzeichnet, dass** das Halteelement (3B, 3C, 3F) mit dem Verbindungselement (2) verbunden, insbesondere einstückig verbunden ist.

4. Verbindungssystem nach Anspruch 1, 2 oder 3, **dadurch gekennzeichnet, dass** das Verbindungselement (2) in das Schlauchelement (1) axial einschiebbar ist und sich im Verbindungszustand wenigstens teilweise radial innerhalb des Schlauchelementes befindet.

5. Verbindungssystem nach einem der Ansprüche 1 bis 4, **dadurch gekennzeichnet, dass** das Verbindungselement (2) in das Leitungselement (3) axial einschiebbar ist und sich im Verbindungszustand wenigstens teilweise radial innerhalb des Leitungselementes befindet.

6. Verbindungssystem nach einem der Ansprüche 1 bis 5, **dadurch gekennzeichnet, dass** die beiden Dichtungen zwischen dem Verbindungselement (2) und dem Leitungselement (3) sowie dem Schlauchelement (1) als Radialdichtungen ausgebildet sind und dass wenigstens eine, insbesondere beide, der Dichtungen des Verbindungselementes (2) mit dem Schlauchelement (1) einerseits und mit dem Leitungselement (3) andererseits durch ein jeweils beide Dichtungspartner von radial außen umfassendes Druckelement (4, 5, 6) radial zusammengepresst wird.

7. Verbindungssystem nach Anspruch 6, **dadurch gekennzeichnet, dass** ein oder mehrere Druckelemente durch eine Fadenwicklung (5, 6) gebildet sind.

8. Verbindungssystem nach einem der Ansprüche 1 bis 7, **dadurch gekennzeichnet, dass** der Schnappring (3B) im Verbindungszustand auf das Element, auf das er aufgeschnappt ist, einen radialen Druck ausübt.

9. Verbindungssystem nach einem der Ansprüche 1 bis 8, **dadurch gekennzeichnet, dass** der Schnappring (3B) im entspannten Zustand einen Innendurchmesser aufweist, der kleiner ist als der Außendurchmesser des Elementes, auf das er aufschnappbar ist.

10. Verbindungssystem nach einem der Ansprüche 1 bis 9, **dadurch gekennzeichnet, dass** dasjenige der Elemente des Verbindungssystems, auf das der Schnappring (3B) aufschnappbar ist, eine außen azimutal umlaufende Rastfläche (2B), hinter der der Schnappring einschnappt, und/oder ein umlaufendes Gewinde (2B) zum Aufschrauben eines weiteren Elementes des Verbindungssystems aufweist.

## Claims

1. Connection system for creating a vascular cannula for connection to a blood vessel, comprising a flexible tube element (1), particularly a graft, a conduit element (3) as well as a connecting element (2) for fluid-tight connection to the tube element on the one side and to the conduit element on the other side, wherein the connecting element axially overlaps with the tube element as well as the conduit element, and wherein a retaining element (3B, 3C, 3F) having a radially elastically expandable snap ring (3B) is provided, which can be beaded radially outwardly over other parts (3C) of the retaining element, and which, in the connected state, can be snapped onto an element of the connection system.

2. Connection system according to claim 1, **characterized in that** the retaining element (3B, 3C, 3F) is connected to the conduit element (3), more particularly being connected thereto as a single piece.

3. Connection system according to claim 1 or 2, **characterized in that** the retaining element (3B, 3C, 3F) is connected to the connecting element (2), more particularly being connected thereto as a single piece.

4. Connection system according to claim 1, 2 or 3, **characterized in that** the connecting element (2) can be slid axially into the tube element (1), and, in the connected state, is at least partially located radially within the tube element.

5. Connection system according to one of claims 1 to 4, **characterized in that** the connecting element (2) can be slid axially into the conduit element (3) and, in the connected state, is at least partially located radially within the conduit element.

6. Connection system according to one of claims 1 to 5, **characterized in that** the two seals between the connecting element (2) and the conduit element (3) as well as the tube element (1) are in the form of radial seals, and that at least one, more particularly both of the seals of the connecting element (2) with the tube element (1) on the one side and with the conduit element (3) on the other side are compressed radially by way of a pressure element (4, 5, 6) that encloses both sealing partners radially from the outside.

7. Connection system according to claim 6, **characterized in that** one or more pressure elements are formed by a thread binding (5, 6).

8. Connection system according to one of claims 1 to 7, **characterized in that** the snap ring (3B), in the connected state, applies a radial force onto the element onto which it is snapped.

9. Connection system according to one of claims 1 to 8, **characterized in that** the snap ring (3B), in the unloaded state, has an inner diameter that is smaller than the outer diameter of the element onto which it can be snapped.

10. Connection system according to one of claims 1 to 9, **characterized in that** the element of the connection system onto which the snap ring (3B) can be snapped comprises an outwardly azimuthally circumferential detent surface (2B), behind which the snap ring snaps in, and/or a circumferential thread (2B) for screwing on a further element of the connection system.

## Revendications

1. Système de liaison pour la réalisation d'une canule vasculaire, pour la liaison à un vaisseau sanguin avec un élément de tuyau flexible (1), en particulier un greffon, un élément de ligne (3) et un élément de liaison (2) pour la liaison étanche aux fluides avec l'élément de tuyau d'une part et avec l'élément de ligne d'autre part, dans lequel l'élément de liaison chevauche axialement aussi bien l'élément de tuyau que l'élément de ligne, et dans lequel un élément de maintien (3B, 3C, 3F) est pourvu d'un anneau encliquetable (3B) apte à être élargi radialement de façon élastique et pouvant être rabattu radialement vers l'extérieur sur d'autres parties (3C) de l'élément de maintien, et lequel peut être encliqueté sur un élément du système de liaison dans l'état relié.

2. Système de liaison selon la revendication 1, **caractérisé en ce que** l'élément de maintien (3B, 3C, 3F) est relié à l'élément de ligne (3), en particulier relié d'une seule pièce.

3. Système de liaison selon la revendication 1 ou 2, **caractérisé en ce que** l'élément de maintien (3B, 3C, 3F) est relié à l'élément de liaison (2), en particulier relié d'une seule pièce.

4. Système de liaison selon la revendication 1, 2 ou 3, **caractérisé en ce que** l'élément de liaison (2) peut être introduit axialement dans l'élément de tuyau (1) et se trouve au moins partiellement radialement à l'intérieur de l'élément de tuyau dans l'état relié.

5. Système de liaison selon l'une des revendications 1 à 4, **caractérisé en ce que** l'élément de liaison (2) peut être introduit axialement dans l'élément de ligne (3) et se trouve au moins partiellement radialement à l'intérieur de l'élément de ligne dans l'état relié.

6. Système de liaison selon l'une des revendications 1 à 5, **caractérisé en ce que** les deux joints d'étanchéité entre l'élément de liaison (2) et l'élément de ligne (3) ainsi que l'élément de tuyau (1) sont conçus comme des joints radiaux, et **en ce qu'**au moins l'un des joints d'étanchéité de l'élément de liaison (2), en particulier les deux, sont comprimés radialement d'une part avec l'élément de tuyau (1) et d'autre part avec l'élément de ligne (3), par un élément de pression (4, 5, 6) entourant respectivement les deux parties d'étanchéité radialement depuis l'extérieur.

7. Système de liaison selon la revendication 6, **caractérisé en ce qu'**un ou plusieurs éléments de pression (4, 5, 6) sont formés par un enroulement de fil (5, 6).

8. Système de liaison selon l'une des revendications 1 à 7, **caractérisé en ce que** dans l'état relié, l'anneau encliquetable (3B) exerce une pression radiale sur l'élément sur lequel il est encliqueté.

9. Système de liaison selon l'une des revendications 1 à 8, **caractérisé en ce que** dans l'état détendu, l'anneau encliquetable (3B) présente un diamètre intérieur inférieur au diamètre extérieur de l'élément sur lequel il peut être encliqueté.

10. Système de liaison selon l'une des revendications 1 à 9, **caractérisé en ce que** celui des éléments du système de liaison sur lequel l'anneau encliquetable (3B) peut être encliqueté, comporte une surface d'encliquetage (2B) extérieure azimutalement périphérique, derrière laquelle s'encliquète l'anneau encliquetable, et/ou un filetage périphérique (2B) pour le vissage d'un autre élément du système de liaison.
